# NEW EUROPEAN PATENT SPECIFICATION

(11) **EP 0 783 514 B2**
(45) Date of publication and mention of the opposition decision: **01.08.2007**
(45) Mention of the grant of the patent: 17.04.2002
(21) Application number: 95932593.7
(22) Date of filing: 29.09.1995
(51) Int. Cl.: C07J 9/00, A61K 31/575

(54) **STEROL COMPOSITIONS FROM PULPING SOAP**
STERIN ZUSAMMENSETZUNGEN AUS HOLZAUFSCHLUSSSEIFE
COMPOSITIONS DE STEROLS OBTENUES A PARTIR DU SAVON ISSU DE LA TRITURATION DE LA PATE A PAPIER

(30) Priority: 29.09.1994 US 314945
(43) Date of publication of application: 16.07.1997
(60) Divisional application: 01118776.2
(73) Proprietor: THE UNIVERSITY OF BRITISH COLUMBIA, Vancouver, British Columbia V6T 1Z3 (CA)
(72) Inventor: NOVAK, Egon, Richmond, British Columbia V6X 3T1 (CA); KUTNEY, James P., Vancouver, British Columbia V6L 3C7 (CA); JONES, Peter J., Ste. Anne-de-Bellevue, Quebec H9X 1S2 (CA)
(74) Representative: Jones, Helen M.M.
(86) International application number: PCT/CA1995/000555
(87) International publication number: WO 1996/010033

(56) References cited:
- EP-A- 0 057 075
- EP-A- 0 430 078
- WO-A-92/19640
- FR-A- 2 470 135
- FR-A- 2 510 118
- FR-A- 2 510 119
- US-A- 3 965 085
- US-A- 4 044 031
- US-A- 4 153 622
- US-A- 4 208 286
- US-A- 4 279 827
- US-A- 4 422 974
- US-A- 5 286 845
- CHEMICAL ABSTRACTS, vol. 121, no. 16, 17 October 1994 Columbus, Ohio, US; abstract no. 182393, JIN Q ET AL 'Extracting phytosterol from sulfate soap using ethyl acetate' & LINCHAN HUAXUE YU GONGYE , vol. 13, no. 1, 1993 pages 51-56,
- CHEMICAL ABSTRACTS, vol. 081, no. 4, 29 July 1974 Columbus, Ohio, US; abstract no. 014899, NEKRASOVA V B ET AL 'Sterol composition of sulfate soap and tall oil of Kotlas Pulp and Paper Complex' & ISPOL'Z. BIOL. AKTIV. VESHCHESTV DEREVA, 1973 pages 44-48,
- TAPPI, vol. 65, no. 2, 1982 NEW YORK US, page 71 UKKONEN K 'Nonvolatile dichloromethane extractives of Gmelina arborea'
- CHEMICAL ABSTRACTS, vol. 091, no. 20, 12 November 1979 Columbus, Ohio, US; abstract no. 159229, ROSHCHIN V I ET AL 'Resinous substances in some Siberian wood species and products from its kraft pulping. 33. Chemical composition of neutral substances of black liquor from pulping of Pinus silvestris' & KHIM. DREV. , no. 4, 1979 LENINGR. LESOTEKH. AKAD.;LENINGRAD; USSR, pages 33-42,
- CHEMICAL ABSTRACTS, vol. 076, no. 26, 26 June 1972 Columbus, Ohio, US; abstract no. 155859, HOLMBOM B ET AL 'Tall oil from pine and birch. II. Unsaponifiable constituents in sulfate soaps and in crude tall oils' & ACTA ACAD. ABO., SER. B , vol. 31, no. 16, 1971 ABO AKAD.;INST. WOOD CHEM. CELLUL. TECHNOL.; ABO; FINLAND, page 18PP
- CHEMICAL ABSTRACTS, vol. 083, no. 22, 1 December 1975 Columbus, Ohio, US; abstract no. 181355, LARINA E I ET AL 'Use of chromatography for analysis of phytosterol produced from the by products of sulfate pulp production' & KHROMATOGR. ANAL. KHIM. DREV. , 1975 pages 323-327,
- CHEMICAL ABSTRACTS, vol. 097, no. 4, 26 July 1982 Columbus, Ohio, US; abstract no. 025316, KRUTOV S M ET AL 'Study of sterols by the chromatographic-mass spectrometric method' & KHIM. MEKH. PERERAB. DREV. DREV. OTKHODOV, vol. 7, 1981 pages 62-65,
- ATHEROSCLEROSIS, vol. 28, 1977 pages 325-338, A. M. LEES ET AL 'Plant Sterols as Cholesterol-Lowering Agents: Clinical Trials in Patients with Hypercholesterolemia and Studies of Sterol Balance'
- PATENT ABSTRACTS OF JAPAN vol. 007 no. 056 (C-155) ,8 March 1983 & JP,A,57 206336 (AJINOMOTO KK) 17 December 1982, & DATABASE WPI Section Ch, Week 8305 Derwent Publications Ltd., London, GB; Class D13, AN 83-10783k & JP,A,57 206 336 (AJINOMOTO KK)
- DATABASE WPI Section Ch, Week 8333 Derwent Publications Ltd., London, GB; Class B05, AN 83-738546 & JP,A,58 116 415 ( RYOSHOKU KENKYU-KAI) , 11 July 1983
- CHEMICAL ABSTRACTS, vol. 093, no. 17, 27 October 1980 Columbus, Ohio, US; abstract no. 160958, TABATA T ET AL 'Hypocholesterolemic activity of phytosterol. II' & YAKUGAKU ZASSHI , vol. 100, no. 5, 1980 pages 546-552,
- Lees et al, Atherosclerosis, 28 (1977), 325-338
- High Technology from Finland, The Finnish Academy of Technical Sciences, P. 12-13
- Römpps Chemie Lexikon 8, Auflage 1987, P. 3871
- Becker et al, The Journal of Pediatrics, Vol. 122, 2, 1993, P 292-296
- Heinemann et al., Atherosclerosis, 61 (1986), 219-223
- Akihisa et al, Naturally Occuring Sterols and Related Compounds from Plants, in Physiology and Biochemistry of Sterols, Ed. Patterson et al, 1991, P. 172, 184-189, 196-201
- Applicant's submission filed on october 6, 1999 with the EPO
- Ling et al, Atherosclerosis, 118 (1995), p. 319-331
- Schlief et al, Atherosclerosis, 30, 1978, 245-248
- Pollak OJ et al, Monographs on Atherosclerosis, 1981
- Pollak OJ, 1985, Pharm. Ther., 177-208
- Ling et al, Life Sciences, 57, 1995, 195-206
- Ntianos et al., Biochemica et Biophysica Acta 1390 (1998), p. 234-244
- Figures of Phytosterols structures
- Dissolution test of phytosterol crystals in rapeseed oil
- Expert opinion on the identification of phytosterols
- Pubmed Abstract, Cardiovasc. Drugs Ther., 2003 Sep.-Nov., 17 (5-6), 443-9
- Denke, The American Journal of Clinical Nutrition, 61 (1995), p.826-831
- Ostlund et al, The American Journal of Clinical Nutrition, 70 (1999), p. 826-831
- Becker et al, Pediatrics 89, 1992, 138-142
- Becker et al. J. Pediatrics, 122, 1993, 292-6 (corresponds to D5)
- Vanhanen et al, Journal of Lipid Research, Volume 34, 1993, 1535-44
- Vanhanen et al., Clinica Chimica Acta, 205, 1992, 97-107
- Weisweiller P. et al, International Journal of Clinical Pharmacology, Therapy and Toxocology, Vol. 22, 1984, P. 204-206
- June 2000, Hayes et al, International Atherosclerosis Conference, Stockholm
- Westrate JA et al., 1998, European Journal of Clinical Nutrition, 198, 52, 334-343
- American Journal of Clinical Nutrition, 2005, 81, 1351-8

## Description

### FIELD OF THE INVENTION

This invention relates to the use of sterol compositions derived from pulping soaps as agents to lower the plasma concentration of low-density lipoprotein-cholesterol.

### BACKGROUND OF THE INVENTION

The direct cause of heart attack and angina is a degenerative process known as atherosclerosis. Atherosclerosis results from a number of integrated inherited (genetic) and environmental factors. The interplay of these factors, of which diet in our civilization appears to be the most important, leads to the development of atherosclerosis. Growth of cholesterol filled atherosclerosis plaques ultimately cuts off blood supply to the heart muscle, or alternately to brain or legs, depending on the location of the plaque in the arterial tree.

One of the major risk factors for atherosclerosis that is potentially modifiable is the level of blood cholesterol. A number of well documented studies have shown that the blood cholesterol level is indeed an important predictor for the risk of heart attack and also for strokes. The relationship between blood concentration of cholesterol and risk of these disorders is continuous (spans across all levels of cholesterol) graded (the higher the level, the more likely the disease) with no apparent threshold (even by lowering so-called low levels, one can further decrease the risk of the disease). For example, in people over 40 years of age, a blood cholesterol level of 7.0 mmol/L presents a risk of coronary artery disease three to four times that associated with levels below 5.0 mmol/L. The relationship becomes especially steep when the levels are above 5.2 mmol/L. For instance, the death rate among men with cholesterol of levels 8.0 mmol/L was almost six times that among men with levels of 4.0 mmol/L. These more recent findings are consistent with earlier studies.

Other large clinical trials have shown clearly that by lowering high cholesterol levels, one can reduce the risk of fatal and non-fatal myocardial infarctions, angina, changes in electrocardiograms and in coronary artery bypass surgery. The best known and the first of these trials was at Lipid Research Clinics at which Coronary Primary Prevention Trials showed that with every 1% reduction in total blood cholesterol level, there was a 2% reduction in the risk of coronary artery disease.

For any long term preventative therapy of hypercholesterolemia to be successful, it has to be commenced at a relatively early age and continue indefinitely. While a low-fat diet is the corner stone of such long term therapy, up to 60% of patients become non-compliant after six months. The difficulty in non-compliance is marked in many Western countries by a general diet which is high in fat. The poor cholesterol profile of many patients is exacerbated by the prevalence of additional risk factors for cardiovascular disease such as high blood pressure, diabetes, obesity and smoking.

Dietary modification as a therapy for atherosclerosis and other cardiovascular diseases has been refined significantly over the past 10 to 15 years. In particular, it has been recognized by researchers that plant sterols (phytosterols) are effective in lowering plasma cholesterol levels: Lees et al. Atherosclerosis, 28 (1977) 325-338; Kudehodkar et al., Atherosclerosis, 23 (1976) 239; Day . Artery, 18(3):125-132 (1991).

Phytosterols are sterol-like compounds synthesised in plants with no nutritional value to humans. In plants they are required for cell function in a manner similar to the way in which cholesterol is required in humans. The average Western diet contains up to 360 mg of phytosterols per day. Recently, these dietary plant sterols have received a great deal of attention because of their possible anti-cancer properties and their ability to decrease cholesterol levels when fed to a number of mammalian species, including humans.

Chemically, phytosterols closely resemble cholesterol in structure. The major phytosterols are beta-sitosterol, campesterol and stigmasterol. Others include stigmastanol (beta-sitostanol), sitostanol, desmosterol, chalinasterol, poriferasterol, clionasterol and brassicasterol. The chemical structures of beta-sitosterol, campesterol and stigmastanol are as follows:

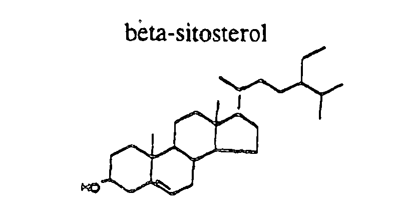

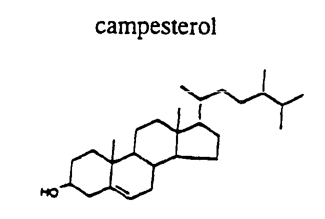

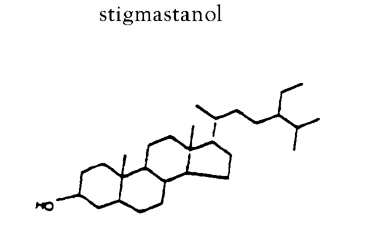

The mechanism by which phytosterols lower blood cholesterol in animals is unclear, but it appears to involve the inhibition of cholesterol absorbtion from the proximum jejunum by competing with cholesterol at specific uptake sites. Research data has also suggested that some phytosterols are not absorbed at the proximal jejunum at all (sitostanol) and, when there is absorbtion (beta-sitosterol), it is in very limited quantities.

Based on these research findings, the use of phytosterols as a dietary supplement to reduce cholesterol absorbtion has been widely investigated. Lees et al., supra; Pollak, Pharmac. Ther., 31 (1985) 177-208; Raicht et al., Biochimica et Biophysica Acta, 388 (1975) 374-384.

In Lees et al., *supra*, a comparison was made between the effects of siterosterol preparations from two sources, soy sterols and tall oil sterols, on plasma cholesterol. Pliant sterol preparations were found to be effective in treating patients with hypercholesterolemia. Pollak, *supra*, is a survey paper of phytosterols and their effect on serum lipids. Raicht, *supra,* describes further the effect of beta-sitosterol on sterol balance and rate-limiting enzymes of sterol metabolism.

It is generally accepted that phytosterols offer a unique combination of long-term safety, efficacy, and versatility in human treatment. The ongoing challenge with respect to phytosterols is in their isolation and purification from plant sources and in determining additional sources which are cost-effective, manageable on a large-scale and which exhibit hypocholesteremic effects.

Traditionally, phytosterols have been isolated from sources such as corn oil, wheat germ oil, soya bean pitch and corn oil pitch. Similarly, tall oil pitch, which is obtained during the process of preparing paper from wood, particularly pine wood, has been used as a phytosterol source. Generally, in this process, wood chips are digested with caustic soda to produce a pulp or "soap". The soap is then distilled to remove the volatile materials leaving a "pitch" as the residue. It is from this pitch that researchers have isolated phytosterols.

There are some marked disadvantages to these traditional sources of phytosterols. The tall oil pitch is an extremely complex material comprising resins, fatty acids, oxidation products, esterified materials and phytosterols. Although the pitch is inexpensive in that it is the tailing left from various manufacturing processes, it is very difficult to recover high molecular weight sterols from it in good yields and at the high purities required for pharmaceutical uses.

United States Patent No. 3,840,570 to Jullan provides a process for preparing sterols from tall oil pitch by extraction in water - alcohol - hydrocarbon mixture followed by saponification and subsequent purification. The starting material in this process is tall oil pitch from which are extracted phytosterols and various impurities. It is recognized that, in any tall oil pitch purification process, the long-chain alcohol and acid impurities are particularly difficult to separate from the sterols (which are, themselves, high molecular weight alcohols).

Other researchers have addressed the issue of sterol purification from tall oil pitch: United States Patent No. 2,835,682 to Steiner and Fritz; United States Patent No. 2,573,891 to Christenson. It is important to note that in each of these known purification processes, the starting material was tall oil pitch which has the recovery problems discussed above.

It is an object of the present invention to obviate or mitigate the above disadvantages.

In US-A-3965085 a soap formed from pulping of wood products is treated to remove non-saponifiable neutral compounds by mixing the soap with ketone and then a water-immiscible solvent such as petroleum hydrocarbons. The solvent phase is separated and solvent recovered therefrom to give a mixture of neutral compounds such as sterols.

### SUMMARY OF THE INVENTION

The process disclosed provides the use of a product in the manufacture of a medication for use in a method of lowering the plasma concentration of low density lipoprotein-cholesterol wherein the product is a phytosterol composition obtainable by a process for purifying and preparing phytosterol compositions from pulping soap which comprises
in a first phase, mixing the pulping soap with a solvent mixture comprising a ketone selected from the group having the general structure RCOR¹ where R and R¹ are alkyl groups, an aliphatic hydrocarbon selected from C₅-C₁₀ hydrocarbons and water and having no alcohol, at a temperature generally from 25°C to 150°C to form a creamy precipitate; and
in a second phase, purifying from the creamy precipitate a phytosterol composition.

The present invention as claimed in claim 1 and claim 7 also provides unique compositions which are effective in preventing or treating dyslipidemias and which comprise beta-sitosterol, campesterol and stigmastanol in which campesterol and stigmastanol together comprise at least 50% of the concentration of beta-sitosterol. The phytosterol compositions provided herein are significantly different from those found in plants, foods and oils. In particular, the provision of stigmastanol appears to enhance the efficacy. These compositions may additionally comprise various co-occurring compounds, which may or may not be phytosterols. In particular, these co-occurring compounds may include tritepenes, long chain alcohols and other alcohol-soluble organic compounds.

The present invention further provides the use of the compositions described herein in the manufacture of medication for use in a method of lowering the plasma concentration of low-density lipoprotein-cholesterol, e.g. to prevent or treat primary and secondary dyslipidemias and atherosclerosis including coronary heart disease, peripheral vascular disease and strokes in humans and animals.

The unique compositions of the present invention have exhibited excellent results in lowering total (TC) and low density lipoprotein (LDL) blood cholesterol. In addition, and quite surprisingly, the compositions of the present invention were found, in different animal species, to maintain or elevate plasma levels of high-density lipoprotein (HDL) blood cholesterol. This feature of the present invention is critically important given the fact that research has shown that, irrespective of TC levels, as the plasma HDL level decreases, the risk of atherosclerosis increases. Phytosterols isolated from tall oil pitch, soybean and other sources have not, to the knowledge of the present inventors, exhibited this unique HDL effect.

It is believed that the surprising effect of the compositions used in the present invention is due, at least partially, to the use of the pulping soap as the starting material and to the unique separation process.

### BRIEF REFERENCE TO THE DRAWINGS:

Various aspects of the invention will be illustrated by the following non-limiting drawings wherein:
Figure 1 represents a bar graph illustrating the effects of Forbes-1 and Forbes-2 on TC concentrations in rats;
Figure 2 represents a bar graph illustrating the effects of Forbes-1 and Forbes-2 on LDL-cholesterol concentrations in rats;
Figure 3 represents a bar graph illustrating the effects of Forbes-1 and Forbes-2 on HDL-cholesterol concentrations in rats;
Figure 4 represents a bar graph illustrating the effects of Forbes-3 on serum TC in hamsters;
Figure 5 represents a bar graph illustrating the effects of Forbes-3 on serum LDL-cholesterol in hamsters;
Figure 6 represents a bar graph illustrating the effects of Forbes-3 on serum HDL-cholesterol in hamsters;
Figure 7 represents a bar graph illustrating the effects of various dietary treatments on cholesterol levels in male and female hamsters;
Figure 8 represents a bar graph illustrating the effects of various dietary treatments on plasma cholesterol levels in male hamsters;
Figure 9 represents a bar graph illustrating the effects of various dietary treatments on plasma cholesterol levels in female hamsters;
Figure 10 represents a bar graph illustrating the effects of various dietary treatments on plasma triglyceride levels in male and female hamsters;
Figure 11 represents a bar graph illustrating the effects of various dietary treatments on HDL/apo-B ratios in male and female hamsters;

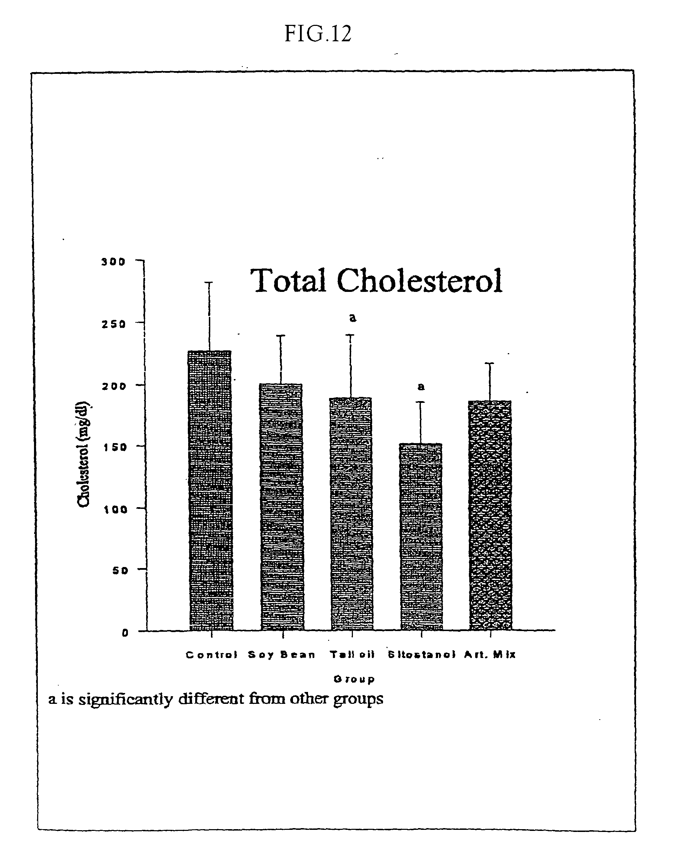

Figure 12 represents a bar graph illustrating the effects of dietary treatments on total cholesterol in hamster 45 day study;

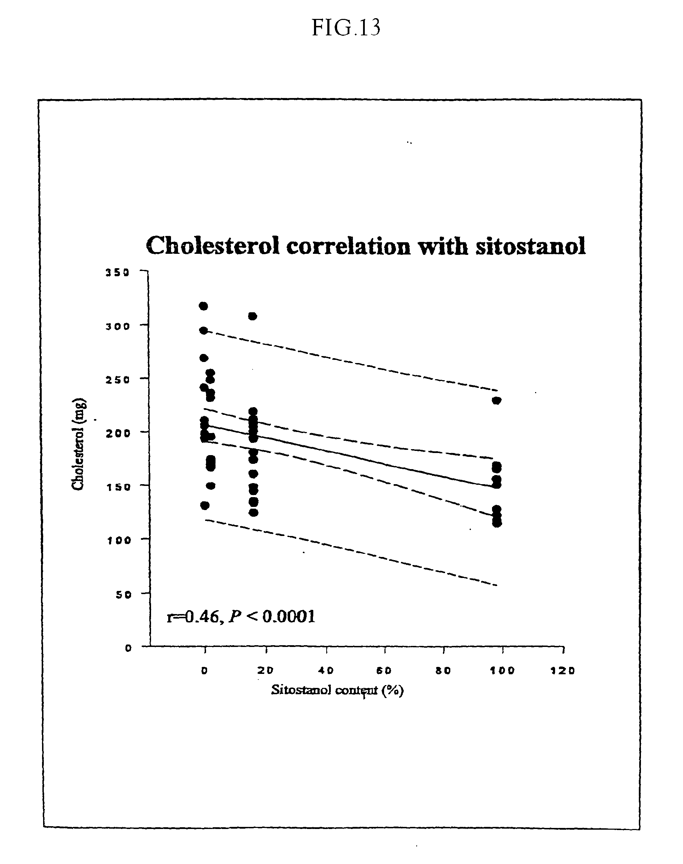

Figure 13 represents a bar graph illustrating the effects of dietary treatments on cholesterol correlation with sitostanol;

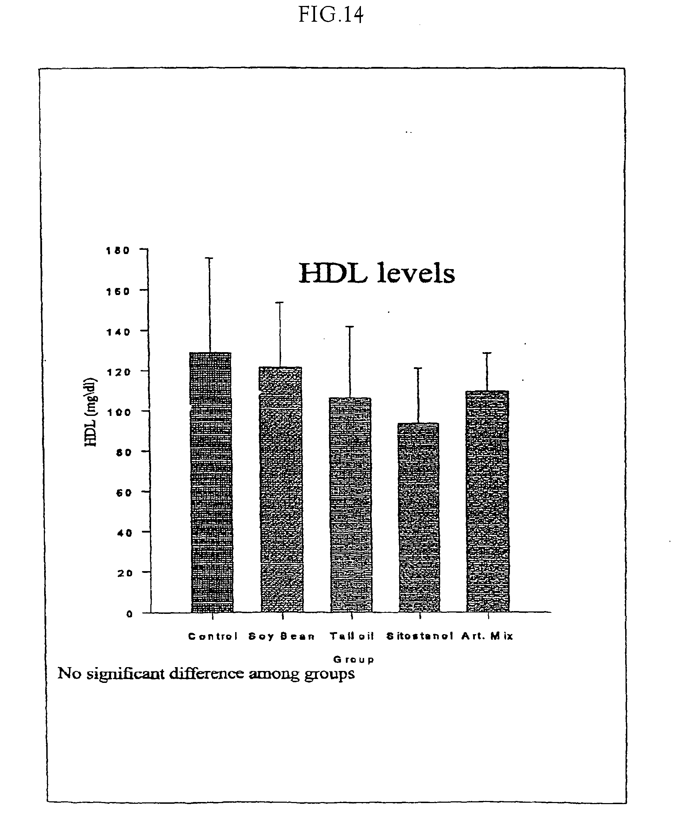

Figure 14 represents a bar graph illustrating the effects of dietary treatments on HDL levels in hamsters over 45 days;

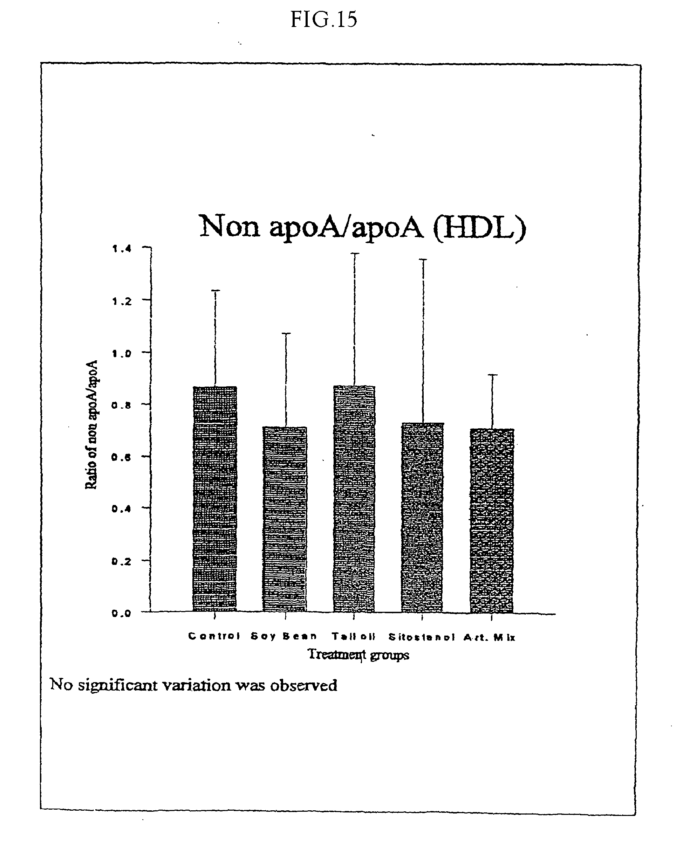

Figure 15 represents a bar graph illustrating the effects of dietary treatments on Non-apoA/apoA ratios in hamsters over 45 days; and

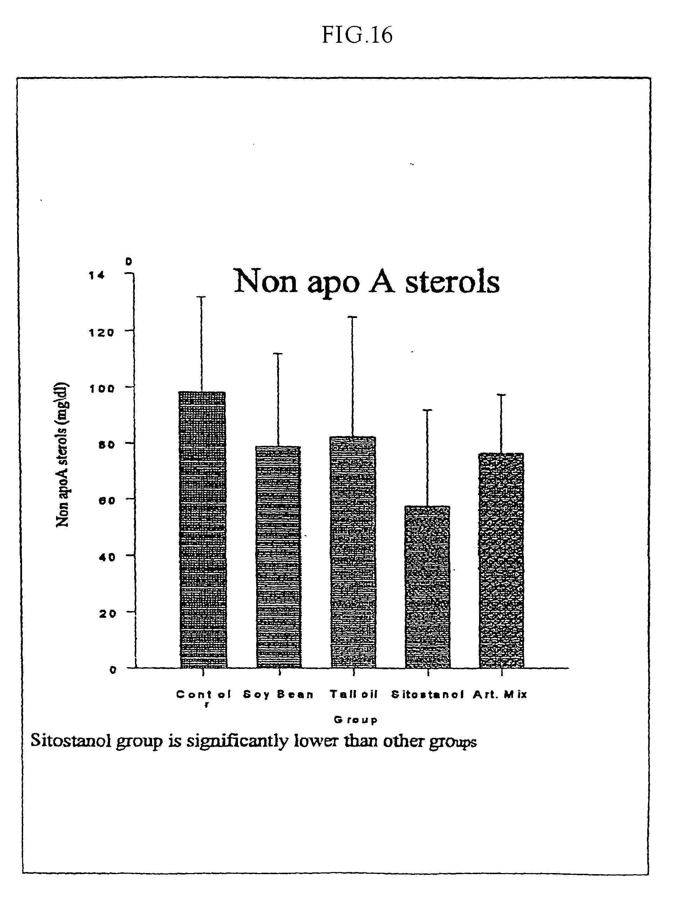

Figure 16 represents a bar graph illustrating the effects og dietary treatments on Non-apoA sterols in hamsters over 45 days.

### PREFERRED EMBODIMENTS OF THE INVENTION

The process for obtaining the compositions used in the present invention comprises the steps of:
(A) obtaining or preparing the starting material, a plant-derived pulping soap;
(B) extracting from the soap a creamy precipitate using an appropriate solvent; and
(C) purifying from the creamy precipitate a phytosterol composition.

There are numerous possible sources of the plant-derived pulping soap. Generally, in a known process (the "Kraft" process) wood chips are treated with caustic soda to produce a soap. The wood chips may be derived from any hard wood or soft wood variety of tree including, but not limited to, fir, cedar, pine, spruce, oak, hemlock and poplar. Most preferably, the chips are derived from any Pacific Northwest American or European forest variety of woods.

In the extraction phase, the soap is mixed with a ketone and water solution. A hydrocarbon solvent is used to extract the sterols. This step is performed at temperatures generally from about 25°C to about 150°C, but most preferably from about 50°C to about 100°C. Most preferably, this extraction phase is continued over 15 to 24 hours. It is important to note that the use of alcohol is neither required nor suggested during the extraction phase. The extraction process of the present invention is conducted using a ketone-water-hydrocarbon solvent.

The ketone is selected from the group having the general structure RCOR¹ where R and R¹ are alkyl groups. Preferably the alkyl groups are C₁ - C₆ groups. Most preferably, the ketone is 2-propanone (acetone). The hydrocarbon is selected from the group comprising all C₅ - C₁₀ hydocarbons. Most preferably, the hydrocarbon is hexane.

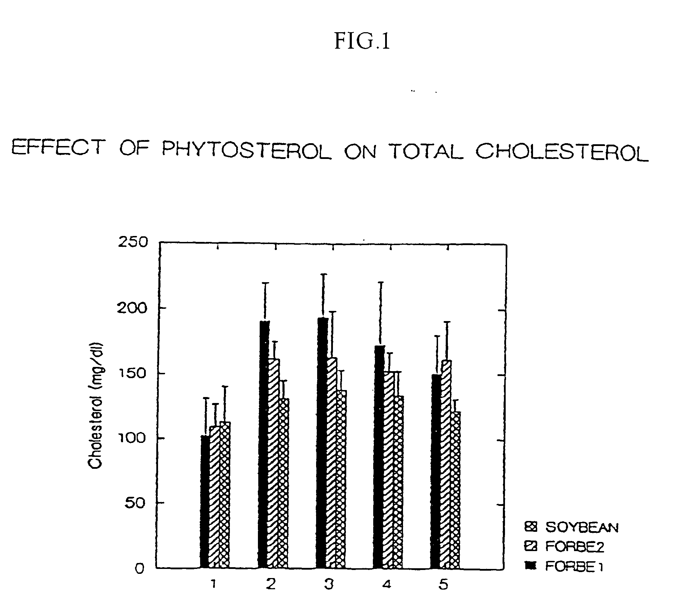

As depicted in Figure 1, the product of the extraction phase is a creamy precipitate or residue from which is purified the phytosterol composition. This purification phase may be conducted by crystallization, chromatographic separation or by any other suitable procedures. Most preferably, the creamy precipitate is dissolved in alcohol, cooled slowly, then filtered and washed with cold alcohol. The residue is dried, and the resultant product is a phytosterol composition.

In a preferred form, the alcohol used in the purification phase is selected from the group having the general structures R-CHOHR, R-CH₂OH, and RCOH where R is a C₁ - C₄ alkyl group. Most preferably, the alcohol is methanol. The cooling phase may be affected at temperatures from 10° Celsius to 0° Celsius, most preferably at 3 to 4° Celsius for 24 hours.

The phytosterol compositions resulting from the processes described herein may be incorporated directly into food supplements and vitamin formulations and into drugs for on-going and preventive treatment of atherosclerosis and its consequences, strokes, heart attacks and peripheral vascular disease. In addition, it is contemplated within one embodiment of the present invention that the phytosterol compositions described herein be provided in the form of medications with suitable adjuvants or carriers. For example, these compositions may be incorporated or prescribed concurrently with selected lipid-lowering agents, to decrease the necessary dosage, and hence the toxicity, of these latter compounds.

The phytosterol compositions used according to the present invention have exhibited a marked ability to modify lipoproteins, even at lower phytosterol concentrations than in known formulations. More surprisingly, however, has been the effect of these compositions on increasing plasma levels of high density lipoproteins (HDL), an effect heretofore not associated with any other tall oil-derived phytosterol composition. It is believed that this unique effect may be due to the use of pulping soaps as the starting material or the provision of stigmastanol as an element of the composition.

According to the present invention the compositions used therein comprise a ratio of phytosterols such that campesterol and stigmastanol together represent at least 50% of the total concentration of betasitosterol, and the following ratio : beta-sitosterol (1); campesterol (0.2-0.4) and stigmastanol (0.2-0.5). The novel compositions of the present invention comprise the following ratio of phytosterols as compared to soybean-derived phytosterols:

| | Ratio of Known Phytosterols | | | |
|---|---|---|---|---|
| | Approximate Purity (%) | B-Sitosterol | Campesterol | Stigmastanol |
| Soybean | | 1 | 0.640 | 0.005 |
| Forbes-1 | 91.0 | 1 | 0.354 | 0.414 |
| Forbes-2 | 77.0 | 1 | 0.330 | 0.203 |
| Forbes-3 | 90.0 | 1 | 0.268 | 0.299 |

The composition and purity of two other extracts within the scope of the present invention are as follows:

| | Composition (%) | | | |
|---|---|---|---|---|
| | Approximate Purity (%) | B-Sitosterol | Campesterol | Stigmastanol |
| Forbes-4 | 99.0 | 62.6 | 16.6 | 23.2 |
| Forbes-5 | 98.3 | 64.7 | 16.4 | 17.2 |

In every composition described herein, there may be additional compounds present which may or may not be phytosterols. For example, it has been found that campestanol, another phytosterol, may be present in a relatively small quantity. In addition, straight chain fatty alcohols, such as behenyl (C22) and lignoceryl alcohol (C24) may be present. In order to determine the nature of these co-occurring compounds, gas liquid chromatography analysis has been conducted on each of the most preferred compositions of the present invention.

Another preferred composition within the scope of the present invention comprises the following components:
Campesterol 14.1%
Campestanol 3.5%
B-sitosterol 62.8%
stigmastanol 16.9%
for a total phytosterol concentration of 97.3 %.

### EXAMPLE 1 - Extraction and Purification

A batch of 3 kg of pulping soap was obtained from B.C. Chemicals Inc. A mixture of 3 L of acetone and 1.5 L of water was prepared to which the soap was added. The mixture was extracted continuously with 4.5 L of hexane at 50°C for 24 hours using an 18 L evaporator. The resultant extraction product was then dried over sodium sulphate and allowed to evaporate. This produced 460 g of residue or creamy precipitate.

The creamy precipitate was warmed and stirred using a magnetic bar and 460ml of methanol was slowly added. The mixture was refluxed under stirring for 15 min. and cooled slowly for 3-5 hours. The mixture was refrigerated at 3-4°C overnight and then filtered and washed (twice) with 150 ml cold methanol. Finally, the mixture was maintained in a vacuum for 2 days yielding 100 g of mixture with a purity of 82% (i.e. 82 g of phytosterols).

### EXAMPLE 2 - Evaluation of the Effects of Phytosterol Compositions in Rats

Ninety male Wistar rats (80-100 g) were divided into 3 experimental modules: Forbes-1 composition; Forbes-2 composition and soybean. The thirty rats within each module were further divided into 5 dietary regimes as indicated in Table 2. The rats were kept on reverse lighting cycle, and fed for 10 days with a basal semi-purified diet (Table 1) supplemented with different amounts of cholesterol and phystosterol (Table 2). Within each of the 5 dietary groups, 2 rats were administered the Forbes-1 composition, 2 rats were administered the Forbes-2 composition and 2 rats were administered soybean-derived phytosterol (Sigma).

**Table 1.**

| Composition of experimental diet | |
|---|---|
| Ingredients | % |
| Casein | 20 |
| Cornstarch | 21.5 |
| Sucrose | 35 |
| Fixed-oil* | 18 |
| D1-methionine | 0.5 |
| Mineral mixture | 4.00 |
| Vitamin mixture | 1.00 |

| | |
|---|---|
| * Safflower and lard mixed in a 1:3 ratio. | |

**Table 2.**

| Dietary regimens | | |
|---|---|---|
| Groups | Sterols added to the basal diet (%) | |
| | Cholesterol | Phytosterol |
| 1 | 0 | 0 |
| 2 | 1 | 0 |
| 3 | 1 | 0.2 |
| 4 | 1 | 0.5 |
| 5 | 1 | 1 |

At the end of the feeding period, the rats were intraperitoneally injected with deuterium oxide (0.4 ml) and deprived of food and water for at least 2 hours. The rats were then anaesthetized with halothane. Blood samples were withdrawn from the heart. Samples of liver, small intestine and muscle were quickly removed, weighed, put in liquid nitrogen and stored at 80°C until determination of cholesterol synthesis. Total cholesterol, LDL and HDL cholesterol were determined with a commercial kit (Biopacific Diagnostic Inc).

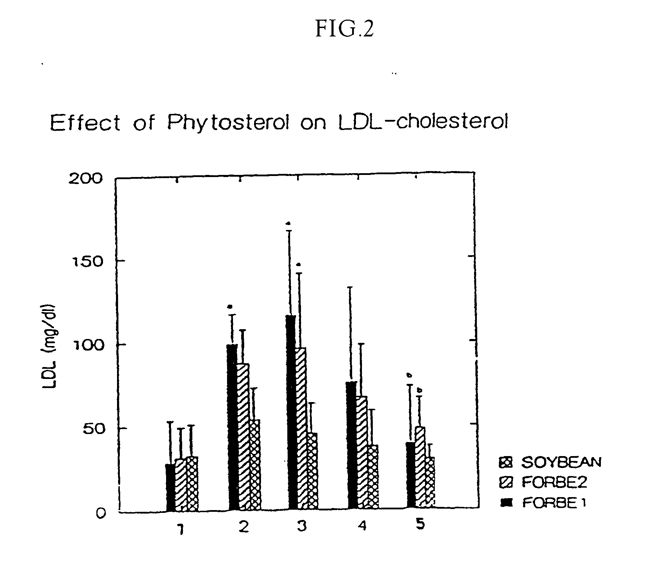

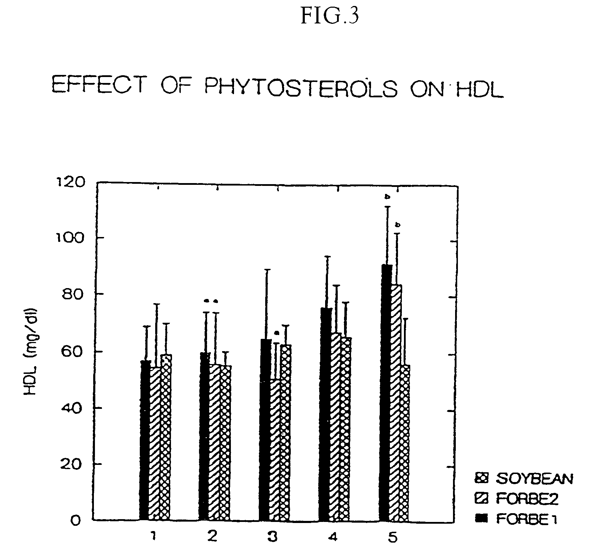

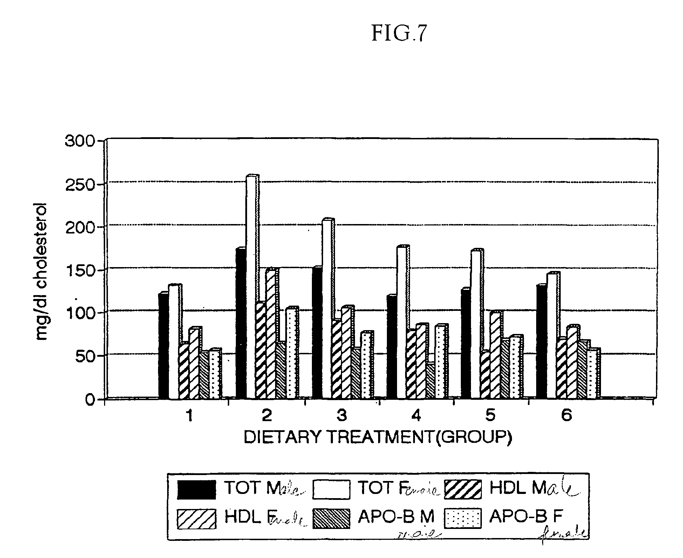

The results of the effects of the phytosterol compositions on total cholesterol, LDL and HDL are represented in Figures 1, 2 and 3 respectively. The efficacy of the Forbes-1 and Forbes-2 is evident from the reduction in LDL-cholesterol shown in Figure 2 and in the increase in HDL-cholesterol shown in Figure 3, particularly by Forbes-1. In Figure 7, the addition of cholesterol (dietary group 2) to the base diet (group 1) resulted in an increase in circulating cholesterol concentrations. Progressive addition of increasing levels of phytosterols (groups 3-5) resulted in a normalization of cholesterol levels in groups fed Forbes-2 and Forbes-1, but not soybean phytosterols, as determined by regression analysis. Figure 3 shows that Forbes-2 and Forbes-1 phytosterols possess better cholesterol - lowering efficacy than the soybean phytosterols for LDL. Figure 3 demonstrates the greater HDL-raising ability of the preferred compositions of the present invention, particularly Forbes-1, compared to the soybean phytosterols.

### EXAMPLE 3 - Evaluation of Effects of Phytosterol Compositions in Hamsters

The present study was to examine the effect of dietary phytosterol compositions of the present invention on the dietary cholesterol-induced elevation of serum cholesterol concentrations in hamsters.

A total of 40 male hamsters (80-100g), housed individually in stainless mesh cages were fed rodent chow and acclimated for three days in an air conditions room (20-22°C, lights on 1700-0500). Hamsters were then divided into five groups of 8 animals each group, and fed for 34 days, a basal semi-purified diet (Table 3) supplemented with different amounts of cholesterol and one of the phytosterol compositions of the present invention (Forbes 3) (Table 4).

**Table 3.**

| Composition of experimental diet | |
|---|---|
| Ingredients | % by weight |
| Casein | 20 |
| Cornstarch | 28 |
| Sucrose | 36.3 |
| Corn oil | 5.0 |
| Cellulose | 5.0 |
| D1-methionine | 0.5 |
| Mineral mixture | 4.00 |
| Vitamin mixture | 1.00 |
| Choline bitartrate | 0.2 |
| Cholesterol | 0.025, 0.25 |

**Table 4.**

| Dietary regimens | | |
|---|---|---|
| Groups | Cholesterol added to control diet | Phytosterol added to control diet |
| | % | % |
| 1 | 0.025 | None |
| 2 | 0.25 | None |
| 3 | 0.25 | 0.25 |
| 4 | 0.25 | 0.5 |
| 5 | 0.25 | 1.0 |

At the end of the feeding period the animals were intraperitoneally injected with deuterium oxide (0.4 ml), and deprived of food and water for at least 2 hours. The hamsters were then anaesthetized with halothane. Blood samples were withdrawn from the heart. Other tissue samples including liver, small intestine and muscle were quickly removed, weighed, put in liquid nitrogen and stored at -80°C until determination of cholesterol synthesis. Total cholesterol, HDL and LDL cholesterol were determined using a commercial kit. The results were statistically evaluated with ONEWAY analysis of variance procedure (SYSTAT).

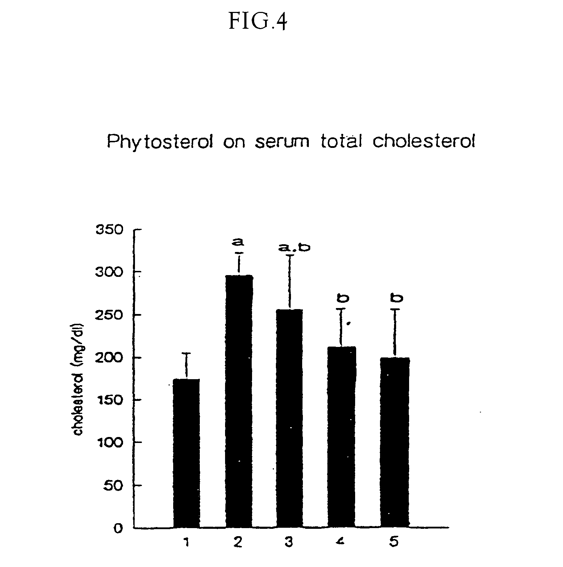

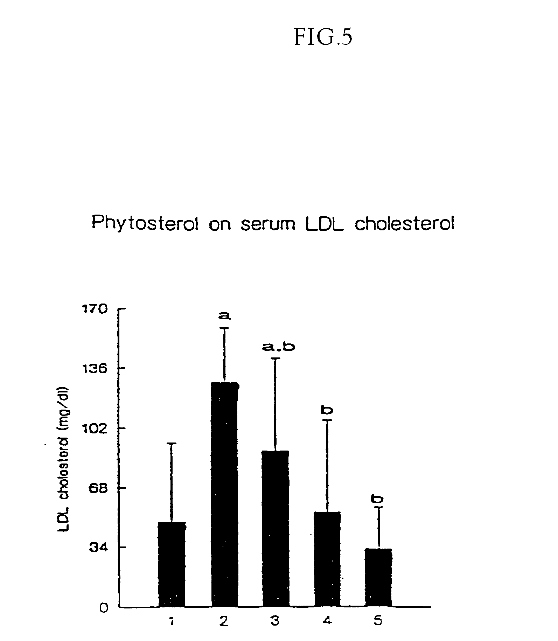

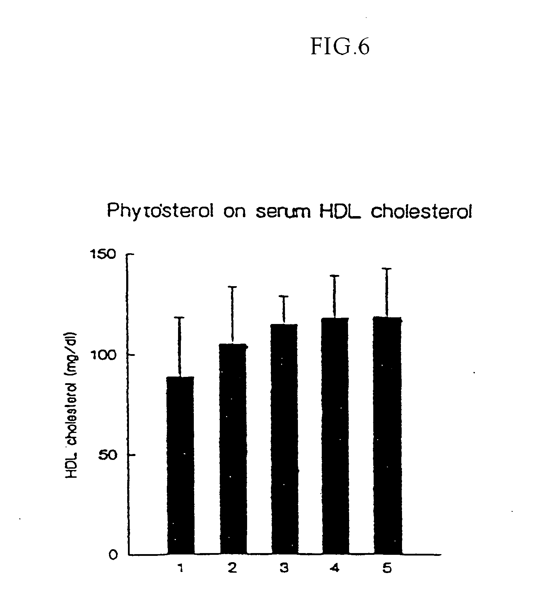

Hamsters fed the high cholesterol diet had significantly higher serum total cholesterol and LDL cholesterol than did those fed the normal cholesterol (0.025%) diet. The supplementation of phytosterol at levels of 0.5% and 1% remarkably abolished these increases induced by high cholesterol consumption (Figures 4 and 5). The LDL cholesterol concentration in group 5 was lower compared to the levels in hamsters fed normal cholesterol-containing diets (Figure 5). Furthermore, there was negative regression association of total cholesterol and LDL cholesterol to the level of phytosterol-added in diet (Figure 6).

Supplementation of phytosterol caused a slight increase in HDL, but without yielding a significant difference (Figure 6).

### EXAMPLE 4 - Evaluation of Phytosterol Composition Effects in Hamsters - 90 day trial

Six groups of 20 hamsters (10 males, 10 females) were fed semi-purified diets containing 30% fat (polyunsaturated/saturated fat ratio = 0.3) for 90 days. Diet 1 was cholesterol free. Diets 2-6 contained 0.25% (wt/wt) dietary cholesterol. Diets 3 and 4 contained Forbes phytosterols (greater than 90% purity) at 0.5 and 1%, respectively. Diets were made from primary ingredients every week. Fat, phytosterol and cholesterol levels were determined by gas liquid chromatography. All animals had free access to water and diets throughout the experimental period. Animals were weighed weekly. Food intake was also determined every day and averaged per week by weighing food cups before and after each 24h feeding period. After 90 days feeding, animals were sacrificed using halothane and blood was collected for lipoprotein profile analyses. Circulating total, apoB containing particles and HDL cholesterol and triglyceride levels were determined. Also, just prior to sacrifice, cholesterol absorption and synthesis rates were determined using 14C-cholesterol disappearance from the gut and deuterium incorporation into tissue cholesterol methods, respectively. Uptake of phytosterols into intestine and other tissues was also examined. In addition, samples of intestine and liver were stored to be provided to Bio-Research Laboratories of Senneville, Quebec, for histopathological, carcinogenicity and enzyme function analyses.

### Results:

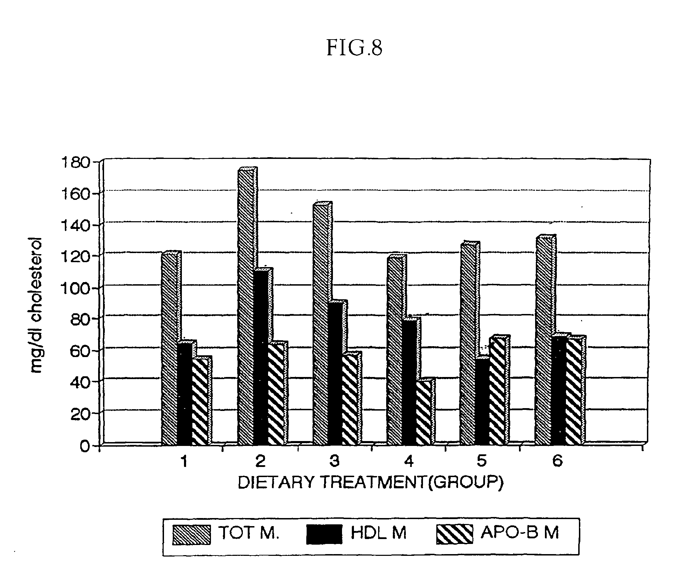

Results are shown in Figures 7-11. Groups in these Figures are often referred to by number, which correspond to those described in Experimental Design. Letters above bars in bar-graphs identify significant differences between groups. Where letters are given, bars sharing the same letter are not significantly different, while those with different letters are different at a statistical level of p<0.05.

Shown in Figure 7-9 are the circulating cholesterol data for male and female hamsters consuming the test diets over 90 days. Significant effects of sex were observed in total circulating cholesterol levels for animals consuming diet 2, the base diet with added cholesterol only, and diet 5 containing cholesterol + soyabean phytosterols. Females, although no different from males in cholesterol levels on the basal diet (group 1), exhibited a higher response to added cholesterol alone compared with males. Adding phytosterols negated this difference, with the exception of group 5.

Cholesterol level data broken down into sexes are shown in Figures 18 and 19. For males (Figure 18), addition of cholesterol alone to the basal diet resulted in a significant increase in total circulatory cholesterol level. Addition of Forbes phytosterols at 0.5 percent resulted in a trend towards a decrease in cholesterol level, however, addition of Forbes phytosterols at 1% elicited a statistically significant reduction in cholesterol, to approximately the same levels of the control group without added cholesterol. When soyabean phytosterols were added to the diet at 0.5 or 1%, there was no significant decrement in circulating total cholesterol level in males. For HDL, a differential effect of Forbes versus soyabean was observed, where Forbes feeding produced no change in the HDL levels of the group given cholesterol alone (group 2), however, feeding soyabean resulted in a significant decline in HDL values at both levels tested (groups 5 and 6). There were no significant differences across apoB containing cholesterol particles, however, there was a trend towards lower levels with the feeding of cholesterol Forbes at 1 %, compared with other groups.

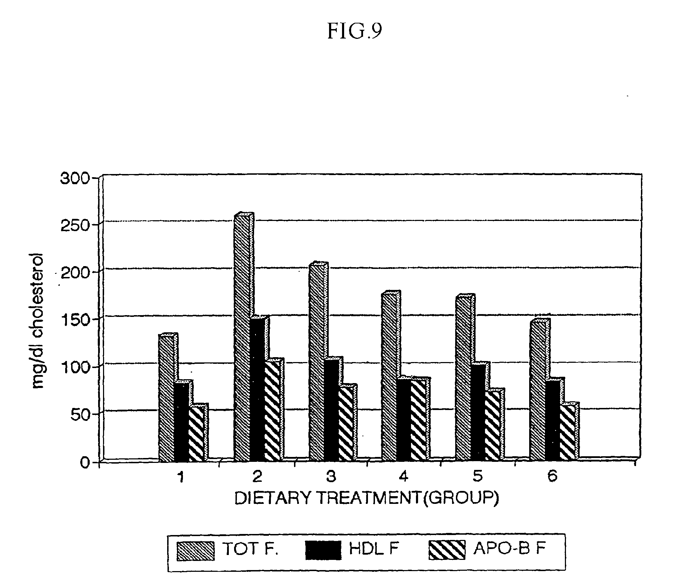

Data for females are shown in Figure 9. For total and HDL cholesterol, there was a pronounced influence of adding dietary cholesterol alone. Addition of either type of phytosterol source at 1% resulted in significant and similar declines in total and HDL cholesterol concentrations. ApoB containing particle levels were not influenced by diet.

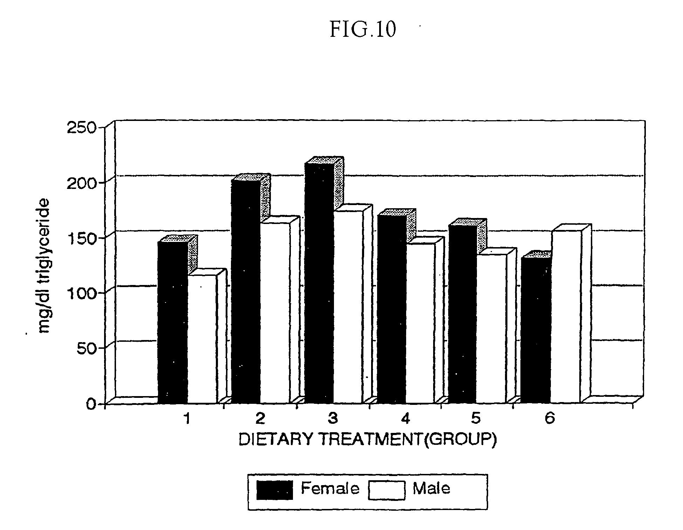

Circulating triglyceride levels in hamsters consuming the test diets for 90 days are shown in Figure 10. There was an increase in circulating triglyceride levels in female animals given the basal diet with cholesterol and Forbes 0.5% compared to the basal diet alone, however, no other inter-group differences were observed in either sex. In males, there was no effect or trend of diet on triglyceride concentrations.

In summary form, the ranking of male results on the test diets are as follows:

**Table 5:**

| | Total Cholesterol | LDL | HDL |
|---|---|---|---|
| Forbes 0.5% | 4 | 2 | 1 |
| Forbes 1.0% | 1 | 1 | 2 |
| Soya 0.5% | 2 | 3-4 | 4 |
| Soya 1.0% | 3 | 4-3 | 3 |

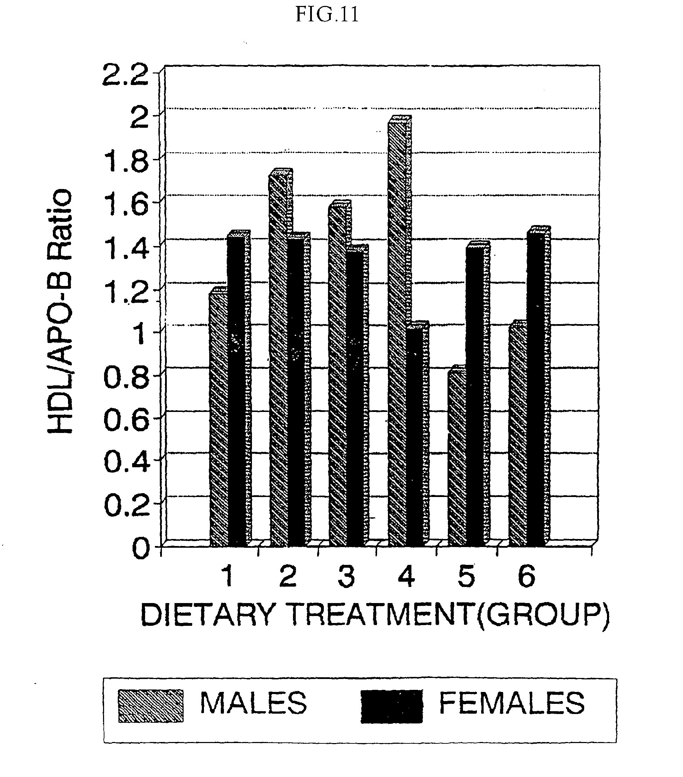

The advantage of the Forbes compositions (those of the present invention) can be clearly seen. In addition, similar rankings were found for the HDL:ApoB ratio in males (Figure 11):

| | |
|---|---|
| Forbes 0.5% | 2 |
| Forbes 0.1% | 1 |
| Soya 0.5% | 4 |
| Soya 1.0% | 3 |

It was also found that the HDL:LDL ratio for the Forbes compositions were almost double those of β-sitosterol alone.

### EXAMPLE 5 - Effects of Phytosterol Composition on Rabbits

In this study, two rabbits were assessed over 43 days with respect to the effects of one of the compositions of the present invention (Forbes 1% in diet) on their total cholesterol profiles. The results are as follows:

**Table 6:**

| Rabbit Total Cholesterol Profiles (mg/dl) | | |
|---|---|---|
| Date | Rabbit A | Rabbit B |
| 14/06/95 | 95 | 215 |
| 18/06/95 | 129 | 162 |
| 26/06/95 | -- | starting feeding with |
| | | Forbes 1% |
| 07/07/95 | 75 | 106 |
| 09/07/95 | -- | starting feeding with |
| | | Forbes 1% |
| 18/07/95 | 90 | 112 |
| 25/07/95 | 82 | 118 |
| 01/08/95 | 95 | 119 |
| 08/08/95 | 76 | 114 |

A decrease in total cholesterol can be seen in both Rabbits A and B during the two weeks of phytosterol composition (Forbes 1%) administration. This effect continued even after the discontinuation of Forbes 1 %. The effects of the compositions of the present invention on total cholesterol lowering linger past the initial administration phase.

### EXAMPLE 6 - Effects of Phytosterol Composition on Apo-E Deficient Mice (Reference)

*Animals:* Nineteen 5-week old male Apo-E deficient mice were purchased from Jackson Laboratory, USA. Animals were randomly divided into 2 groups, 9 animals in control group and 10 mice in experimental Forbes group. After 5 days as an adaptation period mice were bled from tale into capillary tubes and plasma was separated by centrifugation of blood. Mouse plasma lipids were estimated.

*Diet:* Low-fat, low-cholesterol mouse chow was purchased from Jamieson's Pet Food Distributors Ltd., Vancouver, B.C. Tall-oil derived phytosterols were extracted from tall-oil soap, using the process described in the present invention. The purity and percentage of each individual phytosterol in the mixture of final product were assessed by gas liquid chromatography. The final produce showed up to 95 % in purity and contained 69% sitosterol, 15% campesterol and 16% stigmastanol. Mouse chow was ground to a fine powder state. To this powder 0.15% (w/w) cholesterol (Sigma) was added and mixed well. A portion of this cholesterol-supplemented diet was repelleted, dried and used for feeding control group of mice, and another portion of that was supplemented with 2% (w/w) tall-oil extracted phytosterols, repellet, dried and used for feeding of experimental group of mice.

*Biochemical assays:* Plasma total cholesterol and triglyceride were measured using enzymatic kit (Boehringer Mannheim) and HDL-cholesterol was estimated by previously published precipitation method using polyethylene glucol 6000.

*Body weight and food consumption:* Mouse body weight and food consumption were measured weekly.

**Table 7:**

| Mouse mean body weight (g) (only monthly measurements are reported) | | |
|---|---|---|
| Date | Forbes group | Control group |
| 20/06/95 | 21.42 | 21.02 |
| 24/07/95 | 29.82 | 28.00 |
| 22/08/95 | 32.12 | 29.73 |
| 12/09/95 | 34.16* | 30.78 |

| | | |
|---|---|---|
| * p<0.05 | | |

**Table 8:**

| Mouse mean weekly food consumption (g) (only monthly measurements are reported) | | |
|---|---|---|
| Date | Forbes group | Control group |
| 26/06-03/07/95 | 19.61 | 21.21 |
| 01/08-08/08/95 | 32.62* | 29.20 |
| 15/08-22/08/95 | 29.32 | 27.10 |
| 05/09-12/09/95 | 35.56 | 30.25 |

| | | |
|---|---|---|
| * p<0.05 | | |

*Other findings:* No side effects were observed with regard to new diets. All animals from both groups look normal, with normal habits including bowel habit. One mouse in the control group was found dead on 11/07/95. Since the mouse body was not kept, the autopsy was not performed. Another mouse from Forbes group was found dehydrated with body weight lost. The animal was sacrificed and the reason for its sickness was found to be due to malocclusion (teeth overgrowth).

*Statistical analysis:* Results were analyzed using t-test two-samples assuming equal variances.

### Results:

Results up to the present time are summarized in the following Tables.

**Table 9:**

| Mouse mean plasma total cholesterol level (mg/dl). | | |
|---|---|---|
| Date | Forbes group | Control group |
| 20/06/95 | 606.28 | 599.61 |
| 18/07/95 | 1027.96* | 1622.56 |
| 06/09/95 | 1168.57* | 1508.63 |

| | | |
|---|---|---|
| * p<0.0001 | | |

**Table 10:**

| Mouse mean plasma triglyceride level (mg/dl). | | |
|---|---|---|
| Date | Forbes group | Control group |
| 20/06/95 | 110.97 | 120.31 |
| 18/07/95 | 210.17 | 143.71 |
| 06/09/95 | 224.48 | 152.25 |

**Table 11:**

| Mouse mean HDL-cholesterol level (mg/dl). | | |
|---|---|---|
| Date | Forbes group (9) | Control group (7) |
| 08/07/95 | 42.00* | 18.29 |

| | | |
|---|---|---|
| * p<0.001 | | |

It can be seen from the results that the Forbes composition group showed a significant (33%) decrease in total cholesterol, an insignificant increase in triglycerides, and a significant increase in HDL cholesterol (>100%).

### EXAMPLE 7 - Effect of Phytosterol Composition on Hamsters - 45 day Trial

Fifty GS hamsters were accommodated for two weeks in an animal care facility before feeding them a semi-purified diet for 45 days. They were divided into five groups fed 0.25% cholesterol along with one of four mixtures of plant sterols: soy bean, tall oil, pure sitostanol, and artificial mixture representing tall oil phytosterols. The control group received 0.25% cholesterol only. Their food intake was monitored during the study period every three days. Their body weight was measured every week, and at the time of tissue collection. Three days before sacrificing the GS hamsters, they were lightly anaesthetized with diethyl-ether and injected intravenously through the jugular vein with 0.4 ml Intralipid containing 0.18 mg. ¹³C-cholesterol. Directly after the injection, the animals were fed by gavage, 0.6 ml of lipid mixture (coconut, olive, and safflower oils) containing 0.44 mg ¹⁸)-cholesterol. Then, the GS hamsters were kept in their wired cages for 72 hours, provided water and food *ad libitum.*

On the day of sacrificing, each GS hamster was injected i.p., with 1 ml deuterated water, and left for one hour before the killing. The animals were anaesthetized with diethyl ether, and blood samples were collected by cardio-puncture. Liver, gall bladder, small intestine, large intestine, and heart were collected, frozen in liquid nitrogen, and stored in the freezer at -80°C. Their carcasses and faeces were stored at -20°C for further total lipid and sterol analysis.

### Food Intake, Body and Liver Weight Measurements:

GS hamsters food intake was measured every three days. The statistical analysis shows no significant difference among the five groups in their food consumption. The average daily intake in the five groups varied from 8.84 to 9.34g per day, *p*-value = 0.4. The animals showed a significant increase in their body weight of about 25 to 40g during the study period, *p*-value<0.05 (paired t-test). The final measurement of their body weight ranged from 112.5 to 154.3g. No statistical significance was noticed among the different treatment groups, *p*-value = 0.43.

Liver weights varied significantly among the different divisions. The sitostanol treated group showed the lowest liver weights as compared to the control and other groups fed 0.25% cholesterol and different phytosterols respectively. In addition, the soy bean treated group presented similar significant difference to the sitostanol treated one when the data are statistically analyzed using Newman-Keuls test. In general, all groups fed plant sterols showed lower liver weight as compared to control fed 0.25% cholesterol, *p*-value = 0.01. The tall oil sterols and the sitostanol fed GS hamsters had an average liver weight of 15% and 20% less than that of the control, respectively. The natural tall oil and the artificially prepared tall oil demonstrated similar values of liver weights suggesting that the missing compound in the soy bean plant sterol, sitostanol, play a major role in decreasing the sterol content in the liver.

### Lipid Analysis:

### I. Total cholesterol:

Total cholesterol values were determined by using a commercial enzymatic reagent kits on a VP autoanalyzer. Blood samples were measured twice, and the average of the two values was used in the final statistical analysis. One way ANOVA, with Newman-Keuls, and Bonferroni methods were used for the different lipid analysis. Sitostanol decreased significantly total cholesterol level in GS hamsters plasma by 34% as compared to the control. The mean value for control group was 226.9 mg/dl, and the one for sitostanol treated group was 151.2 mg/dl, *p*-value = 0.007. Tall oil phytosterols, and the artificial tall oil mixtures showed similar decrease in plasma cholesterol (17.5%), 118.4 mg/dl and 186.1 mg/dl respectively. However, tall oil phytosterols (Forbes) showed significant decrease in total cholesterol value (175.2 mg/dl) when one off-scale sample value was excluded from the analysis, *p*-value < 0.02 in the tall oil group as compared with the control (0.25 % cholesterol only) group. The correlation between the presence of sitostanol and lower plasma level was significant, *p*-value <0.0001, and r=0.46.

### II. HDL cholesterol:

The apoA portion of the lipoproteins present in the HDL cholesterol did not show any significant changes in their values among the five different groups, *p*-value = 0.18. A non significant decrease of 15% in the mean HDL value in the sitostanol treated group was observed. Nevertheless, this element did not affect the significant decrease in the total cholesterol which was of 34%. The ratio of the non apoA lipoproteins to the apoA (HDL) lipoproteins did not vary significantly among the groups. The confounding effect of the non-significant lower values of HDL in the sitostanol and tall oil groups contributed in creating a non significant result in the non apoA/apoA (HDL) values. In general, the different types of phytosterols did not vary the HDL cholesterol level in GS hamsters plasma.

### III. LDL and/or non apoA sterols:

Sitostanol was efficient in decreasing the non apoA sterols in the plasma. A 55 % decrease in the non apoA lipoproteins was shown, *p*-value = 0.02. Similarly to their effect on total cholesterol, tall oil and artificial mixture tall oil decreased the non apoA sterols by 21% respectively, again suggesting of a strong correlation existing between sitostanol content in the phytosterols and their beneficial effects in decreasing cholesterol levels in the GS hamsters plasma. However, this decrease was not statistically significant due to the variability in the triglyceride values.

### IV. Triglyceride:

When applying the one way ANOVA method on the TG values, they did not pass the normality test. GS hamsters were sacrificed in a non fasting condition (important status for the future cholesterol synthesis, kinetics, and absorption analysis). Because of such situation, different values were off scale. With ANOVA on Ranks, the TG levels showed no statistical difference among the groups. Plant sterols did not affect the plasma TG levels in GS hamsters.

**Table 12:**

| Potency Rank | | | | |
|---|---|---|---|---|
| | Total Cholesterol | HDL | LDL | TG |
| Control | C | C | C | C |
| Soybean β-sitostanol | 4 | 1 | 3-4 | 1-4 |
| Tall oil (Forbes) | 2-3 | 2-2 | 2-3 | 1-4 |
| Sigmastanol | 1 | 4 | 1 | 1-4 |
| Artificial tall oil | 3-2 | 3-2 | 4-3-2 | 1-4 |

In conclusion, the use of the tall oil soap-derived composition according to the present invention exhibited the most favourable profile due to the increase in HDL cholesterol and decrease in total cholesterol. This HDL effect was not seen in the artificial tall oil composition. Similarly, the overall effect of sitostanol is not favourable due to the significant decrease in HDL.

Although it is not entirely clear, it does appear that, with respect to plant sterols, the relatively hydrophobic sterols inhibit more total cholesterol absorption while the relatively hydrophilic sterols have more influence on the level of HDL. The phytosterol compositions of the present invention are unique in that both of these effects are preserved.

## Claims

1. Use of cholesterol-lowering composition comprising beta-sitosterol, campesterol and further comprising stigmastanol, in which campesterol and stigmastanol together comprise at least 50% of the concentration of beta-sitosterol and wherein the ratio of beta-sitosterol is 1.0, campesterol is between 0.2 and 0.4 and stigmastanol is between 0.2 and 0.5 in the manufacture of a medication for use in a method of lowering the plasma concentration of low density lipoprotein-oholesterot.

2. Use according to claim 1 wherein the ratio of beta-sitosterol is 1.0, and either
a) campesterol is 0.354 and stigmastanol is 0.414 or
b) campesterol is 0.330 and stigmastanol is 0.203, or
c) campesterol is 0.268 and stigmastanol is 0.299.

3. Use according to claim 1 or claim 2 in which the method is for prevention or treatment of primary and secondary dislipidemias and atherosclerosis.

4. Use according to any of claims 1 to 3 in which the composition comprises phytosterols and co-occurring compounds selected from triterpenes, long chain alcohols and other alcohol-soluble organic compounds.

5. A medication comprising beta-sitosterol, campesterol and further comprising stigmastanol, in which campesterol and stigmastanol together comprise at least 50% of the concentration of beta-sitosterol and wherein the ratio of beta-sitosterol is 1.0, campesterol is between 0.2 and 0.4 and stigmastanol is between 0.2 and 0.5, and a pharmaceutically effective carrier therefor.

6. A medication according to claim 5 wherein the ratio of beta-sitosterol is 1.0, and either
a) campesterol is 0.354 and stigmastanol is 0.414 or
b) campesterol is 0.330 and stigmastanol is 0.203, or
c) campesterol is 0.268 and stigmastanol is 0.299.

7. A composition comprising beta-sitosterol, campesterol and further comprising stigmastanol, in which campesterol and stigmastanol together comprise at least 50% of the concentration of beta-sitosterol and wherein the ratio of beta-sitosterol is 1.0, and either
a) campesterol is 0.354 and stigmastanol is 0.414 or
b) campesterol is 0.330 and stigmastanol is 0.203, or
c) campesterol is 0.268 and stigmastanol is 0.299.

## Patentansprüche

1. Verwendung einer Cholesterol senkenden Zusammensetzung umfassend beta-Sitosterol, Campesterol und ferner umfassend Stigmastanol, worin Campesterol und Stigmastanol zusammen mindestens 50 % der Konzentration an beta-Sitosterol ausmachen und wobei der Anteil von beta-Sitosterol 1,0 beträgt, der von Campesterol zwischen 0,2 und 0,4 liegt und der von Stigmastanol zwischen 0,2 und 0,5 liegt, bei der Herstellung eines Medikaments für die Verwendung bei einem Verfahren zur Senkung der Plasmakonzentration von Lipoprotein geringer Dichte-Cholesterol.

2. Verwendung nach Anspruch 1, wobei der Anteil von beta-Sitosterol 1,0 beträgt und entweder
(a) der von Campesterol 0,354 beträgt und der von Stigmastanol 0,414 beträgt oder
(b) der von Campesterol 0,330 beträgt und der von Stigmastanol 0,203 beträgt, oder
(c) der von Campesterol 0,268 beträgt und der von Stigmastanol 0,299 beträgt.

3. Verwendung nach Anspruch 1 oder Anspruch 2, wobei das Verfahren der Prävention oder Behandlung von primärer und sekundärer Dyslipidämie und Arteriosklerose dient.

4. Verwendung nach einem der Ansprüche 1 bis 3, wobei die Zusammensetzung Phytosterole und zusammen damit vorkommende Verbindungen, ausgewählt unter Triterpenen, langkettigen Alkoholen und anderen alkohollöslichen organischen Verbindungen, enthält.

5. Medikament, umfassend beta-Sitosterol, Campesterol und ferner umfassend Stigmastanol, wobei Campesterol und Stigmastanol zusammen mindestens 50 % der Konzentration an beta-Sitosterol ausmachen, und wobei der Anteil von beta-Sitosterol 1,0 beträgt, der von Campesterol zwischen 0,2 und 0,4 liegt und der von Stigmastanol zwischen 0,2 und 0,5 liegt; und einen pharmazeutisch wirksamen Träger dafür.

6. Medikament nach Anspruch 5, wobei der Anteil von beta-Sitosterol 1,0 beträgt und entweder
(a) der von Campesterol 0,354 beträgt und der von Stigmastanol 0,414 beträgt oder
(b) der von Campesterol 0,330 beträgt und der von Stigmastanol 0,203 beträgt, oder
(c) der von Campesterol 0,268 beträgt und der von Stigmastanol 0,299 beträgt.

7. Zusammensetzung, umfassend beta-Sitosterol, Campesterol und ferner umfassend Stigmastanol, wobei Campesterol und Stigmastanol zusammen mindestens 50 % der Konzentration an beta-Sitosterol ausmachen und wobei der Anteil von beta-Sitosterol 1,0 beträgt und entweder
(a) der von Campesterol 0,354 beträgt und der von Stigmastanol 0,414 beträgt oder
(b) der von Campesterol 0,330 beträgt und der von Stigmastanol 0,203 beträgt, oder
(c) der von Campesterol 0,268 beträgt und der von Stigmastanol 0,299 beträgt.

## Revendications

1. Utilisation d'une composition diminuant le cholestérol comprenant du béta-sitostérol, du campestérol et comprenant en outre du stigmastanol, dans laquelle le campestérol et le stigmastanol représentent ensemble au moins 50 % de la concentration du béta-sitostérol et dans laquelle le rapport du béta-sitostérol est 1,0, celui du campestérol est entre 0,2 et 0,4 et celui du stigmastanol est entre 0,2 et 0,5, dans la fabrication d'un médicament utile dans une méthode de diminution de la concentration plasmatique du cholestérol-lipoprotéines de basse densité.

2. Utilisation selon la revendication 1, dans laquelle le rapport du béta-sitostérol est 1,0 et
a) celui du campestérol est 0,354 et celui du stigmastanol est 0,414 ou
b) celui du campestérol est 0,330 et celui du stigmastanol est 0,203 ou
c) celui du campestérol est 0,268 et celui du stigmastanol est 0,299.

3. Utilisation selon la revendication 1 ou la revendication 2, dans laquelle la méthode est destinée à la prévention ou au traitement des dyslipidémies primaire et secondaire et de l'athérosclérose.

4. Utilisation selon l'une quelconque des revendications 1 à 3, dans laquelle la composition comprend des phytostérols et des composés co-occurrents choisis parmi les triterpènes, les alcools à longue chaîne et d'autres composés organiques solubles dans les alcools.

5. Médicament comprenant du béta-sitostérol, du campestérol et comprenant en outre du stigmastanol, dans lequel le campestérol et le stigmastanol représentent ensemble au moins 50 % de la concentration du béta-sitostérol et dans lequel le rapport du béta-sitostérol est 1,0, celui du campestérol est entre 0,2 et 0,4 et celui du stigmastanol est entre 0,2 et 0,5 et un support pharmaceutiquement efficace.

6. Médicament selon la revendication 5, dans lequel le rapport du béta-sitostérol est 1,0 et
a) celui du campestérol est 0,354 et celui du stigmastanol est 0,414 ou
b) celui du campestérol est 0,330 et celui du stigmastanol est 0,203 ou
c) celui du campestérol est 0,268 et celui du stigmastanol est 0,299.

7. Composition comprenant du béta-sitostérol, du campestérol et comprenant en outre du stigmastanol, dans laquelle le campestérol et le stigmastanol représentent ensemble au moins 50 % de la concentration du béta-sitostérol et dans laquelle le rapport du béta-sitostérol est 1,0, et
a) celui du campestérol est 0,354 et celui du stigmastanol est 0,414 ou
b) celui du campestérol est 0,330 et celui du stigmastanol est 0,203 ou
c) celui du campestérol est 0,268 et celui du stigmastanol est 0,299.
